# EUROPEAN PATENT APPLICATION

(11) **EP 4 358 542 A1**
(43) Date of publication of application: **24.04.2024**
(21) Application number: 22824540.3
(22) Date of filing: 02.03.2022
(51) Int. Cl.: H04R 25/00, A61B 5/12

(54) **INFORMATION PROCESSING METHOD, INFORMATION PROCESSING SYSTEM, DATA COLLECTION METHOD, AND DATA COLLECTION SYSTEM**

(30) Priority: 18.06.2021 JP 2021101401
(71) Applicant: Sony Group Corporation, Tokyo 108-0075 (JP)
(72) Inventor: MAKINO, Kenichi, Tokyo 108-0075 (JP); MATSUMOTO, Kyosuke, Tokyo 108-0075 (JP); NAKAMURA, Osamu, Tokyo 108-0075 (JP); TSUCHIYA, Shinpei, Tokyo 108-0075 (JP)
(74) Representative: 2SPL Patentanwälte PartG mbB
(86) International application number: PCT/JP2022/008743
(87) International publication number: WO 2022/264540

(57) **Abstract**

An information processing method of an information processing system (200,201) according to the present disclosure includes an adjustment manipulation accepting step and a feedback output step. The adjustment manipulation accepting step accepts adjustment manipulation of the sound output from the sound output unit. The feedback output step estimates the auditory capacity of the user corresponding to the adjustment manipulation from the adjustment manipulation and the signal data of the sound corresponding to the adjustment manipulation, and outputs the feedback.

## Description

### Field

The present disclosure relates to an information processing method, an information processing system, and a data collecting method, and a data collecting system. Background

There is an apparatus that causes a user to listen to an environmental sound of an external environment in a suitable manner by adjusting a parameter of an external-sound capturing function by a head-mounted acoustic device such as a hearing aid, a sound collector, and an earphone (e.g., see Patent Literature 1).

Meanwhile, for example, it is common that the user of the head-mounted acoustic device visits a medical institution or a hearing aid shop only after the user notices that the user has auditory disorder due to the indication of the user or the surrounding person and starts wearing the hearing aid.

### Citation List

### Patent Literature

Patent Literature 1: WO 2016/167040 A

### Summary

### Technical Problem

However, the user cannot notice his/her auditory disorder until his/her auditory capacity decreases to some extent, and does not wear a hearing aid until then, and thus, quality of life (QOL) cannot be improved.

Therefore, the present disclosure proposes an information processing method, an information processing system, and a data collecting method, and a data collecting system capable of improving QOL of a user.

### Solution to Problem

An information processing method of an information processing system according to the present disclosure includes an adjustment manipulation accepting step and a feedback output step. The adjustment manipulation accepting step accepts adjustment manipulation of the sound output from the sound output unit. The feedback output step estimates the auditory capacity of the user corresponding to the adjustment manipulation from the adjustment manipulation and the signal data of the sound corresponding to the adjustment manipulation, and outputs the feedback.

### Brief Description of Drawings

FIG. 1 is a diagram schematically illustrating an information processing system according to an embodiment of the present disclosure.
FIG. 2 is a diagram schematically illustrating an information processing system according to an embodiment of the present disclosure.
FIG. 3 is a diagram illustrating a specific example of learning data collected from a sound output device according to an embodiment of the present disclosure.
FIG. 4 is a diagram illustrating a specific example of learning data collected from a sound output device according to an embodiment of the present disclosure.
FIG. 5 is a diagram illustrating a data collecting system according to an embodiment of the present disclosure.
FIG. 6 is an explanatory diagram of AI learning according to an embodiment of the present disclosure.
FIG. 7 is an explanatory diagram of AI learning according to an embodiment of the present disclosure.
FIG. 8 is an explanatory diagram of AI learning according to an embodiment of the present disclosure.
FIG. 9 is an explanatory diagram of a future auditory capacity prediction AI according to an embodiment of the present disclosure.
FIG. 10A is a flowchart illustrating an example of processing executed by a processing unit according to an embodiment of the present disclosure.
FIG. 10B is a flowchart illustrating an example of processing executed by a processing unit according to an embodiment of the present disclosure.
FIG. 11 is an explanatory diagram of AI learning according to an embodiment of the present disclosure.
FIG. 12 is a diagram schematically illustrating a data collecting method utilizing a television according to an embodiment of the present disclosure.
FIG. 13 is a diagram schematically illustrating a data collecting system utilizing a television according to an embodiment of the present disclosure.
FIG. 14 is a diagram schematically illustrating an information processing system according to an embodiment of the present disclosure.

### Description of Embodiments

Hereinafter, embodiments of the present disclosure will be described in detail with reference to the drawings. Note that, in each of the following embodiments, the same parts are denoted by the same reference signs, and redundant description will be omitted.

### [1. Schematic Information Processing System]

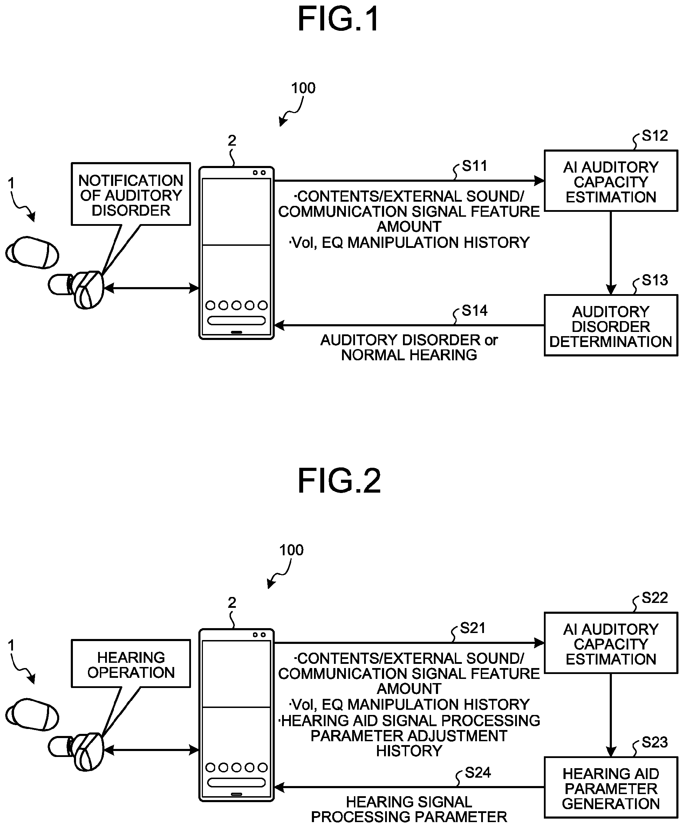

As illustrated in FIG. 1, an information processing system 100 according to an embodiment includes a sound output device 1 and a terminal device 2 including a manipulation device and a processing device. The sound output device 1 is, for example, a wireless earphone and has a sound collecting function and a hearing aid function. The sound output device may be a device other than a wireless earphone as long as the device has a function of outputting sound. Furthermore, the sound output device 1 has a microphone for acquiring user utterance and peripheral sound. The sound output device 1 may have a noise canceling function that reduces the peripheral sound audible to the user wearing the sound output device 1 by outputting sound having a reverse phase to the peripheral sound acquired by the microphone.

Note that the manipulation device and the processing device are parts of the terminal device 2. Therefore, the manipulation device and the processing device in the following embodiment can be replaced with a manipulation unit and a processing unit of the terminal device 2 instead of being separate components.

Furthermore, the manipulation unit and the processing unit may be provided in the sound output device 1. In this case, the sound output device 1 includes a sound output unit that outputs sound, a manipulation unit having a function similar to that of the manipulation device described later, and a processing unit having a function similar to that of the processing device described later.

The terminal device 2 is, for example, a smartphone. The terminal device 2 includes an application program for reproducing music contents, and transmits a sound signal of the music contents to be reproduced to the sound output device 1 to cause the sound output device 1 to output the sound signal.

Further, the manipulation device of the terminal device 2 receives an adjustment manipulation of a voice output from the sound output device 1. The manipulation device is realized by, for example, a touch panel display. The manipulation device receives an adjustment manipulation of the volume (hereinafter, it may be referred to as "Vol") of the sound output from the sound output device 1, an equalizer (hereinafter, it may be referred to as "EQ") that adjusts the sound quality of the sound output from the sound output device 1, and the like.

The processing device of the terminal device 2 estimates the auditory capacity of the user by a learning model (hereinafter, it may be referred to as "AI") that estimates the auditory capacity of the user from the signal data of the sound when the user of the sound output device 1 manipulates the manipulation device and the manipulation device is manipulated, and outputs the feedback to the user.

For example, in a case where the contents of music are output from the sound output device 1, the processing device acquires information such as the contents, the external sound, the signal feature amount, the manipulation history of Vol, the manipulation history of EQ, and the like. Furthermore, for example, in a case where the terminal device 2 has a call function, the processing device also acquires sound of a call (Step S11).

Then, the processing device estimates the auditory capacity of the user by the AI from the information acquired (Step S12), performs auditory disorder determination as to whether or not the user has auditory disorder (Step S13), and presents a notification (auditory disorder or normal hearing) based on the result of the auditory disorder determination to the user as a feedback (Step S14), thereby encouraging the user to wear a hearing aid.

At this time, the processing device may notify the user of whether the user has auditory disorder or normal hearing as the feedback, or may notify the user of that the auditory capacity has decreased, or may notify the user of the estimated auditory capacity (e.g., an audiogram).

In this manner, the information processing system 100 can notify the user of the decrease in auditory capacity before the user or a person around the user notices the decrease in auditory capacity of the user. As a result, the user can notice auditory disorder at an early stage, and, for example, quality of life (QOL) is improved by wearing a hearing aid.

Furthermore, as illustrated in FIG. 2, the processing device of the terminal device 2 can acquire an adjustment history of hearing aid signal processing parameters in addition to the information acquired in Step S11 of FIG. 1 (Step S21). In this case, the processing device estimates the auditory capacity of the user by AI, similar to the example illustrated in FIG. 1 (Step S22).

Thereafter, the processing device generates hearing aid parameters in accordance with the estimated auditory capacity (Step S23). Then, the processing device outputs the generated hearing aid parameter as feedback to the sound output device 1 (Step S24). The hearing aid parameter is a parameter for adjusting the hearing aid function of the sound output device.

As a result, in a case where a decrease in auditory capacity of the user is detected, the sound output device 1 can provide the user with an easily audible reproduction sound by performing signal processing corresponding to a hearing aid. Therefore, the user can benefit from the necessary hearing aid function from the stage before the auditory disorder progresses. Furthermore, according to the information processing system 100, since it is possible to estimate whether or not the user has auditory disorder indirectly from the manipulation history of the manipulation device that is the user interface, it is possible to reduce the burden on the user as compared with a case where the user takes the auditory capacity test.

### [2. Data Collecting System]

For example, the present embodiment may be applied in a case where it is assumed that a user whose auditory capacity has deteriorated "when it is difficult to hear the sound, the manipulation device is manipulated to change the volume and the EQ setting value," to a reproduction sound from the sound output device 1.

At this time, the AI may be caused to estimate the auditory capacity of the user on the basis of "change in volume or EQ" that is a user action on sound that has caused listening difficulty," and may be collected at least as learning data in association with each other.

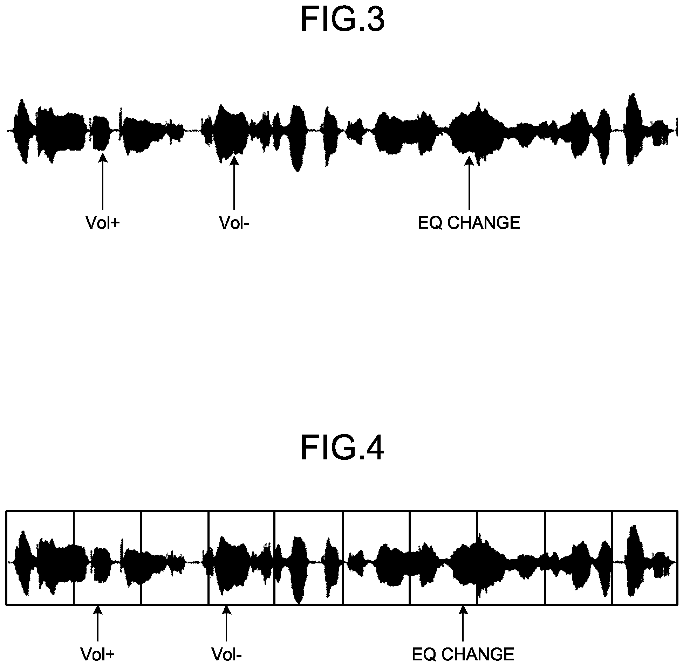

FIGS. 3 and 4 illustrate specific examples of learning data collected from the sound output device. FIG. 3 illustrates recording of waveform data of a sound heard by the user, a manipulation event for the manipulation device, and occurrence of the manipulation event. Herein, when the waveform data itself is recorded, the data amount becomes enormous, and thus, for example, as illustrated in FIG. 4, the sound may be divided into frames, and the feature amount may be extracted and used instead of the waveform data.

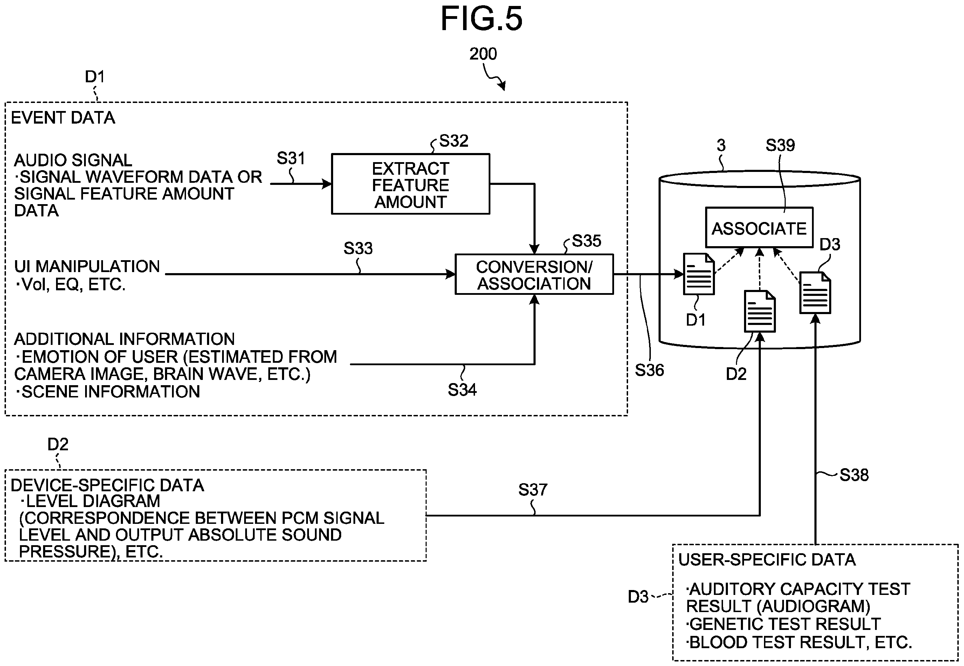

FIG. 5 is an entire view of the data collecting system according to the present embodiment. Note that, although not illustrated in FIG. 5, a data collecting system 200 includes a sound output unit (sound output device 1) that outputs the aforementioned sound, a manipulation unit (manipulation device) that receives an adjustment manipulation of the sound output from the sound output unit, and a data collecting unit that collects manipulation data of the manipulation unit by the user of the sound output unit and signal data of the sound when the manipulation unit is manipulated. As illustrated in FIG. 5, the data collected by the data collecting system 200 has roughly three categories, event data D1, device-specific data D2 and the user-specific data D3, and all or some of these data are accumulated.

The data collecting system 200 collects an audio signal from the sound output device 1 or the terminal device 2 as the event data D1 (Step S31). The audio signal includes signal waveform data or signal feature amount data of sound.

Moreover, the data collecting system 200 collects a user interface (UI) manipulation from the terminal device 2 as the event data D1 (Step S33). The UI manipulation uses manipulation data such as Vol and EQ.

Further, the data collecting system 200 collects additional information as the event data D1 (Step S34). The additional information is situation data of the user when the manipulation device is manipulated by the user. The additional information includes, for example, user's emotion estimated from a camera image, user's brain waves, and the like, and scene information indicating a situation in which the user is in.

The data collecting system 200 converts each data collected as the event data D1 into a predetermined data format, associates the data with the predetermined data format (Step S35), and accumulates the data in a storage device 3 (Step S36). Moreover, the data collecting system 200 collects a level diagram (correspondence between the PCM signal level and the output absolute sound pressure, and the like) of the sound output device 1 as the device-specific data D2 and accumulates the level diagram in the storage device 3 (Step S37).

In addition, the data collecting system 200 collects, as the user-specific data D3, an auditory capacity test result (audiogram) of the user, a genetic test result affecting auditory capacity, a blood test result, and the like, and accumulates the results in the storage device 3 (Step S38).

Then, the data collecting system 200 stores the event data D1, the device-specific data D2 and the user-specific data D3 accumulated in the storage device 3 in association with each other (Step S39).

### [3. AI Learning]

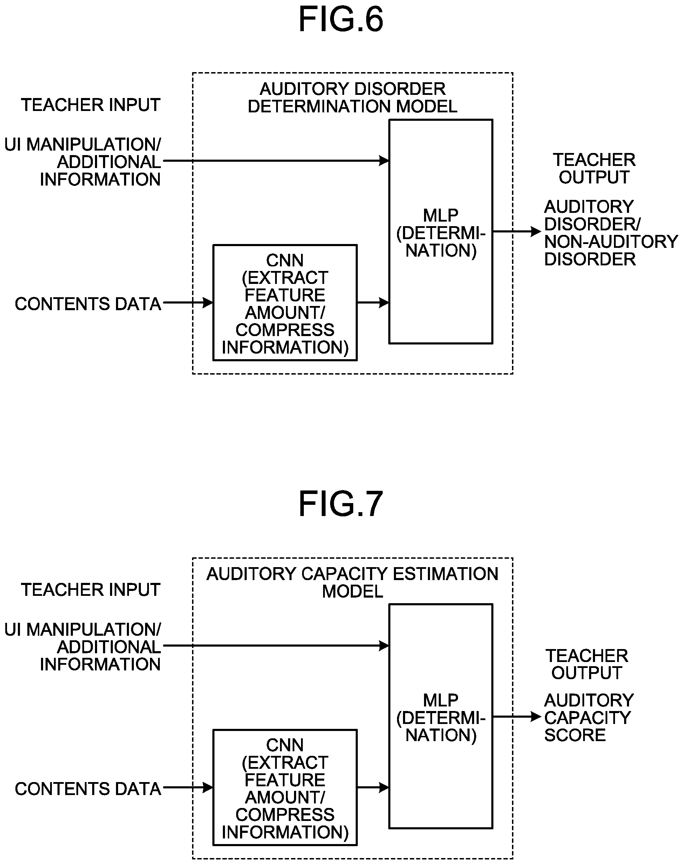

For example, by performing learning by classifying the user into two classes, auditory disorder/non-auditory disorder, using the audiogram information, it is possible to constitute an auditory disorder determination model capable of classifying the user into auditory disorder or non-auditory disorder as illustrated in FIG. 6.

To create teacher output, for example, a user having information on an audiogram is gathered, and a 0/1 label of whether or not the user has auditory disorder is attached from the audiogram using a quartile method. Once learning is completed, auditory disorder/non-auditory disorder is obtained as an output of the auditory disorder determination model for a data input equivalent to the teacher input.

Furthermore, for example, it is possible to constitute an auditory capacity estimation model that estimates the auditory capacity of the user by calculating a score in accordance with the auditory capacity as a numerical value instead of dividing the user into two classes, auditory disorder/non-auditory disorder, and then performing learning using the auditory capacity score as a teacher output as illustrated in FIG. 7. In this case, when the auditory capacity score obtained as the output of the auditory capacity estimation model is equal to or less than a certain threshold, it is determined as auditory disorder, and when the score exceeds the threshold, it is determined as non-auditory disorder.

Then, in a case where the value indicating the estimated auditory capacity level of the user is equal to or less than the threshold, the processing device presents a notification for changing the hearing aid parameter to the user, and changes the hearing aid parameter on the basis of the corresponding to the notification.

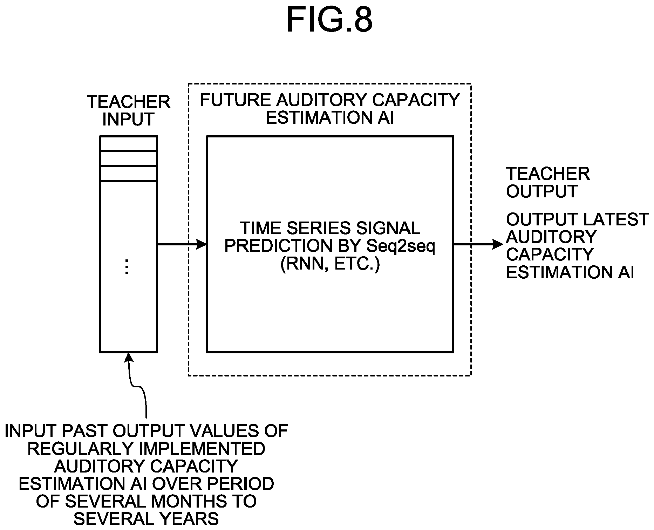

In addition, for example, as illustrated in FIG. 8, the above-described auditory capacity estimation model is periodically performed to collect results, and then learning is performed with the latest auditory capacity estimation result as a teacher output and the past series for a certain period as a teacher input, so that it is possible to constitute the future auditory capacity estimation AI that predicts future auditory capacity for a certain period.

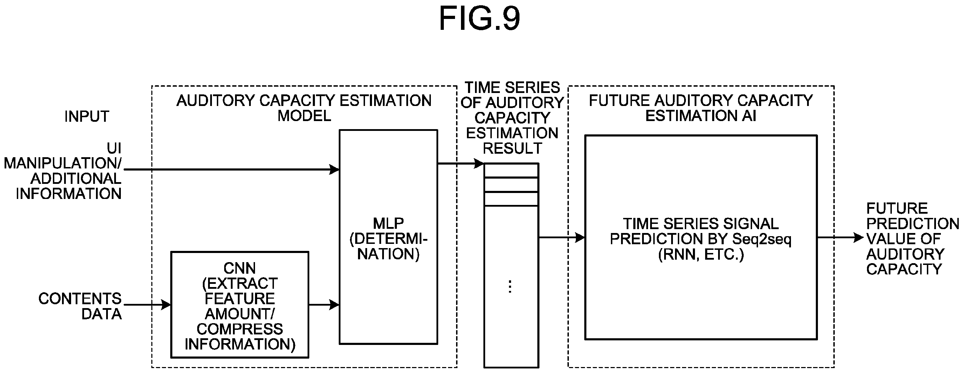

By using this, for example, as illustrated in FIG. 9, it is possible to create the future auditory capacity prediction constant AI including the auditory capacity estimation model and the future auditory capacity estimation AI. Therefore, in a case where the auditory capacity level obtained as the future prediction value of the auditory capacity indicates auditory disorder, it is possible to prompt the user to perform an action change to reconsider the Vol setting by issuing a warning, and to help preventing auditory disorder.

Moreover, since the cause of auditory capacity deterioration is considered to genetic predisposition, damage to the inner ear caused by blood flow disorders, and the like, the auditory capacity prediction reflecting the risk factors possessed by the user can be performed by recording the genetic test result, the blood test result, and the like as "user-specific data " similarly to the auditory capacity test result as risk factors, clustering the user for each risk factor, and then learning the future auditory capacity estimation AI illustrated in FIG. 8 for each cluster.

### [4. Activation of Hearing Aid Function by Updating Sound Output Device]

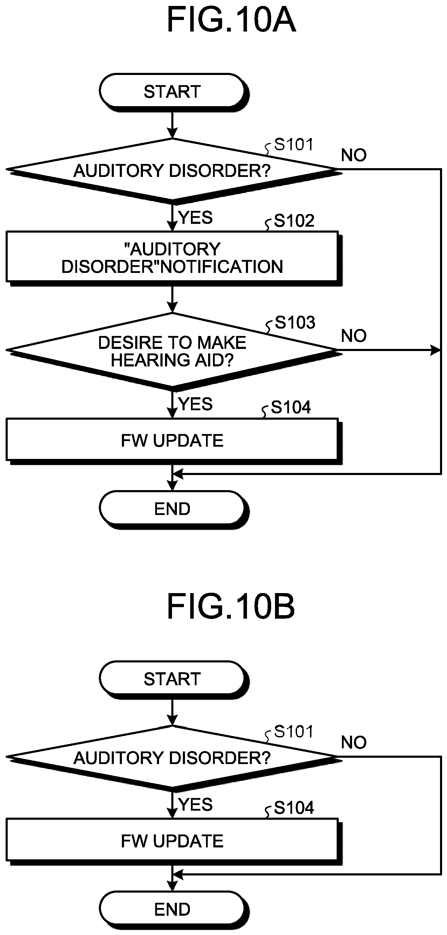

Next, an example of processing executed by the processing device according to the embodiment will be described. As illustrated in FIG. 10 A, the processing device determines whether or not the user has auditory disorder by AI (Step S101), and ends the processing in a case where it is determined that the user does not have auditory disorder (Step S101, No).

Furthermore, in a case where it is determined that the user has auditory disorder (Step S101, Yes), the processing device notifies the user of the auditory disorder (Step S102), and inquires of the user whether or not the user desires to make the sound output device 1 a hearing aid (Step S103). Then, in a case where the user does not desire to make the sound output device 1 a hearing aid (Step S103, No), the processing device ends the processing.

Moreover, in a case where the user desires to make the sound output device 1 a hearing aid (Step S103, Yes), the processing device updates the firmware (FW) of the sound output device 1 to enable a function corresponding to the hearing aid of the sound output device 1 (Step S104), and ends the processing.

Note that, in a case where the sound output device 1 is not authenticated as a medical device, the processing device does not notify the user of "auditory disorder" when notifying the user, but performs notification of indirect expression such as "it seems difficult to hear sound" in Step S102.

Further, for example, as illustrated in FIG. 10B, the processing device determines whether or not the user has auditory disorder by AI (Step S101). In a case where it is determined that the user does not have auditory disorder (Step S101, No), the processing is ended. In a case where it is determined that the user has auditory disorder (Step S101, Yes), the processing device can also end the processing by updating the firmware (FW) of the sound output device 1 to enable the function corresponding to the hearing aid of the sound output device 1 (Step S104) without notifying the user.

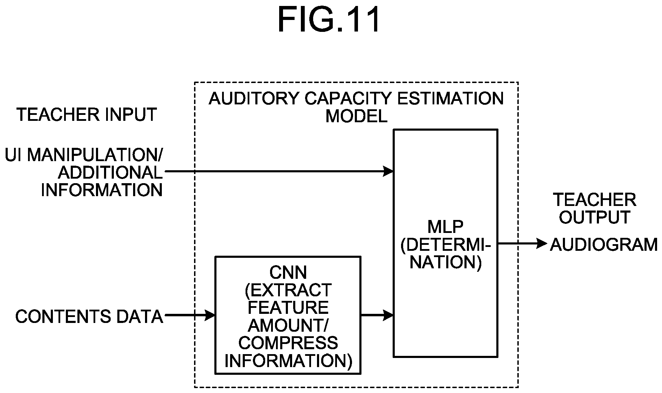

Further, the processing device needs to perform hearing aid fitting in accordance with the auditory capacity of the user when updating to the hearing aid by the processing illustrated in FIG. 10A or FIG. 10B. In this case, as illustrated in FIG. 11, the processing device performs learning using the audiogram as a teacher output, and constitute an auditory capacity estimation model that estimates the audiogram itself.

The processing device can provide the user with a hearing aid function in which appropriate hearing aid parameters are set by estimating an audiogram of the user on the device by the auditory capacity estimation model illustrated in FIG. 11 and generating a fitting formula using the audiogram.

### [5. Example of Utilizing Television]

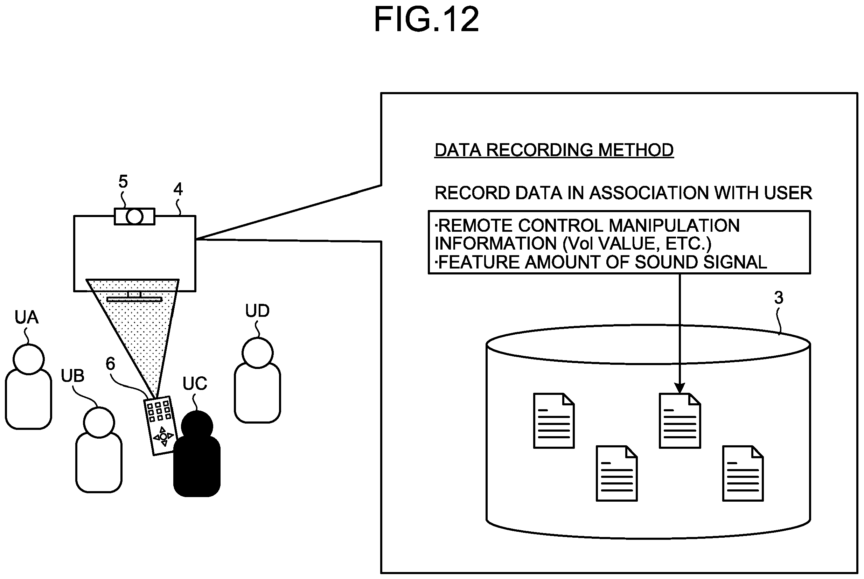

FIG. 12 schematically illustrates a data collecting method utilizing a television. As illustrated in FIG. 12, in the present example, a user is identified using a camera 5 installed in a television 4, and the volume is optimized for a user UC who is watching the television 4.

In optimizing the volume, when there is one user UC who is watching the television 4, it is only required to simply optimize the volume to the user. However, in a case where there is a plurality of users, when the volume is optimized to a specific user UC, there is a possibility that other users UA, UB and UD may be dissatisfied.

Therefore, in particular, in a case where there is a variation in auditory capacity of the users UA, UB, UC and UD, it is desirable to combine with sound field control for performing volume optimization for each surrounding space of the user, or to perform cooperative operation with other devices such as reproducing television sound from devices at hand of the users UA, UB, UC and UD with poor auditory capacity.

Regarding the collection of learning data in the television 4, unlike a sound output device 1, it is not possible to assume that a specific individual exclusively uses the sound output device 1, and it is necessary to identify the users UA, UB, UC and UD for each occurrence of an event such as volume adjustment and then record the user and various data in association with each other. Therefore, in order to identify the users UA, UB, UC and UD to perform data recording, for example, a means for identifying the user such as the camera 5 is used.

In the case of the sound output device 1, the earphone is assumed, whereas in the case of the television 4, the reproduction by the speaker (an example of the sound output device) is basic. Therefore, the sound pressure reaching the users UA, UB, UC and UD depends on the transmission characteristics of the room and the distance between the television and the user.

Therefore, a method is conceivable in which information for estimating the transmission characteristic of the television 4 installation location is registered in addition to the level diagram in the "device-specific data," and the distance between the television 4 and the user is recorded as additional information to the learning data.

In this case, the material of the floor or the wall and the size of the room are estimated from the image of the room captured by the camera 5, the distances between the users UA, UB, UC and UD and the television 4 are similarly estimated from the face image, and the estimation results are integrated to estimate the absolute sound pressure level at the user position.

The trigger for recording the learning data may be a UI manipulation of a remote control (an example of the manipulation device) 6 of the television 4 or the like, or a change in the expression of the user may be used as the trigger when the camera 5 is used as the user identifying means. Furthermore, not only the expression change is simply treated as an event, but also whether the users UA, UB, UC and UD are satisfied or dissatisfied may be estimated from the face expression and recorded as the additional information on the learning data.

In particular, there is a case where an elderly person, a child or the like does not normally manipulate the remote control 6 at all. Therefore, by estimating whether or not there is auditory disorder from attribute information such as a change in expression or age and collecting learning data only in a case where the probability of auditory disorder is high, data collection can be performed more effectively than in a case where only the UI manipulation is used as a trigger.

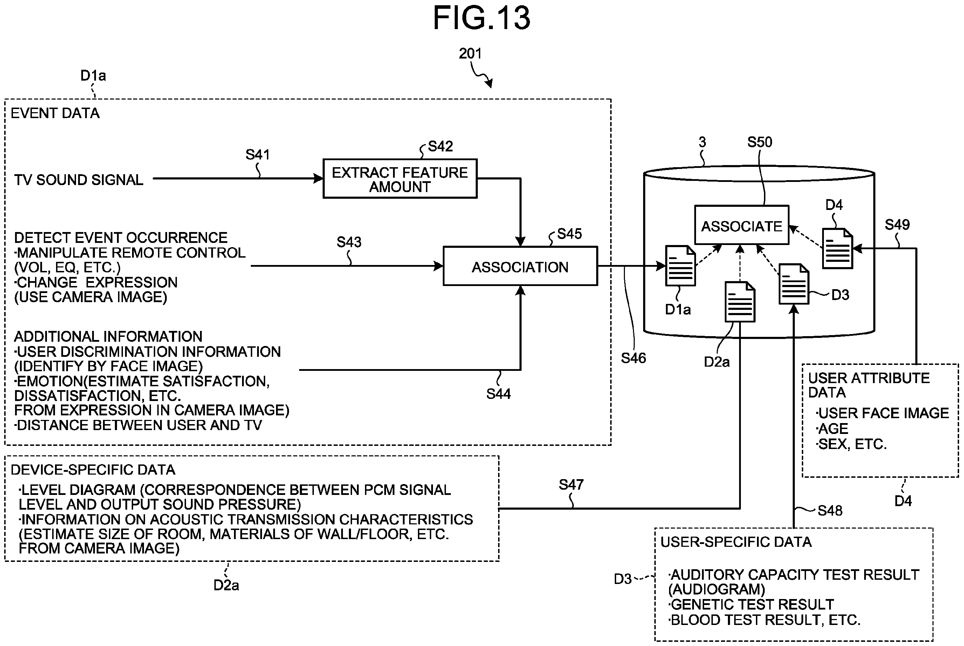

FIG. 13 illustrates a data collecting system 201 utilizing a television. As illustrated in FIG. 13, in the present example, since data recording is performed for a plurality of users UA, UB, UC and UD, the user attribute data D4 is registered.

Then, for example, the data collecting system 201 is different from the data collecting system illustrated in FIG. 5 in that after the users UA, UB, UC and UD are identified by a camera 5 or the like, data of another category to be collected and the user attribute data D4 are collected in association with each other.

The data collecting system 201 collects a television sound signal as event data D1a (Step S41) and extracts the feature amount (Step S42). Moreover, when detecting the occurrence of the event, the data collecting system 201 collects the manipulation of the remote control 6 (e.g., Vol, EQ, and the like), the expression changes of the users UA, UB, UC and UD (using a camera image), and the like as event data D1a (Step S43).

Furthermore, the data collecting system 201 collects additional information as the event data D1a (Step S44). The additional information includes, for example, user identification information, emotions of the users UA, UB, UC and UD, and information on distances from the users UA, UB, UC and UD to the television 4.

The user identification information is identified from, for example, a face image captured by the camera 5. For example, the emotions of the users UA, UB, UC and UD such as satisfaction or dissatisfaction are estimated from the expression of the face image captured by the camera 5. The distances from the users UA, UB, UC, and UD to the television 4 are also estimated from the image captured by the camera 5.

Then, the data collecting system 201 converts each data collected as the event data D1a into a predetermined data format, associates the data with the predetermined data format (Step S45), and accumulates the data in a storage device 3 (Step S46).

Moreover, the data collecting system 201 collects information regarding the acoustic transmission characteristics of the space in which the television 4 is installed in addition to the level diagram (correspondence between the PCM signal level and the output absolute sound pressure, and the like) as device-specific data D2a, and accumulates the information in the storage device 3 (Step S47). For example, the information regarding the acoustic transmission characteristics is acquired by estimating the size of the room where the television 4 is installed, the materials of the wall and the floor, and the like from the image captured by the camera 5.

Furthermore, the data collecting system 201 collects, as user-specific data D3, an auditory capacity test result (audiogram) of the user, a genetic test result affecting auditory capacity, a blood test result, and the like, and accumulates the results in the storage device 3 (Step S48).

Further, the data collecting system 201 collects, for example, information such as face images, ages, and sex of the users UA, UB, UC and UD as user attribute data D4 and accumulates the information in the storage device 3 (Step S49).

Then, the data collecting system 201 stores the event data D1a, the device-specific data D2a, the user-specific data D3, and the user attribute data D4 accumulated in the storage device 3 in association with each other (Step S50).

### [6. Information Processing System]

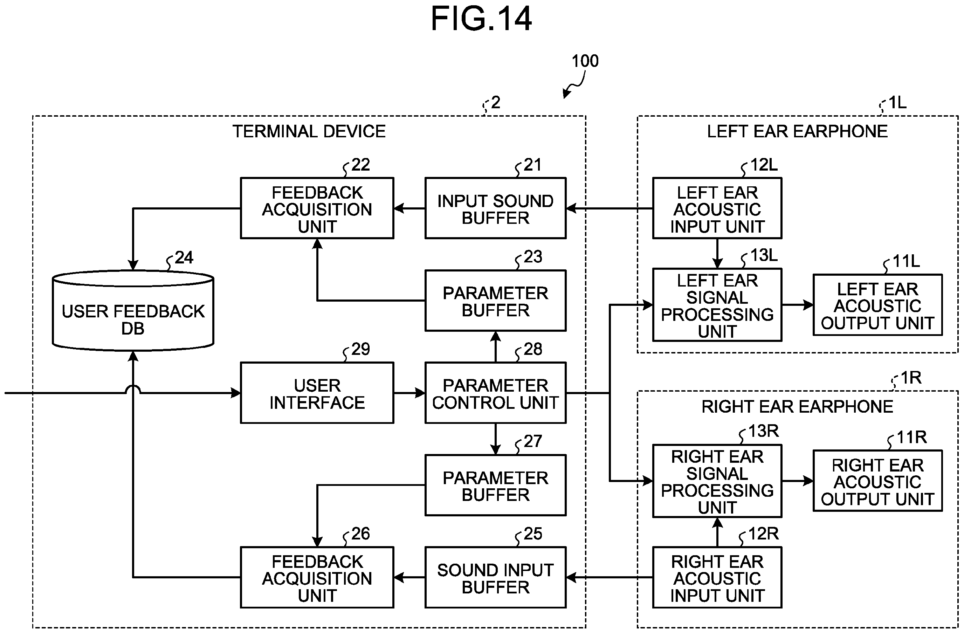

FIG. 14 illustrates a constitution of an information processing system according to the present embodiment. As illustrated in FIG. 14, an information processing system 100 includes a left ear earphone 1L and a right ear earphone 1R and a terminal device 2. The left ear earphone 1L and the right ear earphone 1R correspond to the sound output device 1 illustrated in FIGS. 1 and 2.

The terminal device 2 includes input sound buffers 21 and 25, feedback acquisition units 22 and 26, parameter buffers 23 and 27, a parameter control unit 28, a user feedback DB (database) 24 and a user interface 29.

The user interface 29 functions as the aforementioned manipulation device according to the embodiment. The parameter control unit 28 functions as the aforementioned processing device according to the embodiment. The user feedback DB24 corresponds to the storage device 3 illustrated in FIGS. 5, 12 and 13.

The left ear earphone 1L includes a left ear acoustic output unit 11L, a left ear acoustic input unit 12L and a left ear signal processing unit 13L. The right ear earphone 1R includes a right ear acoustic output unit 11R, a right ear acoustic input unit 12R and a right ear signal processing unit 13R.

The left ear earphone 1L and right ear earphone 1R transmit the input sound to terminal device 2. The terminal device 2 stores the received sound together with the time stamp in the input sound buffers (e.g., 60 Sec each of left and right circular buffers) 21 and 25. This communication may be always performed, or may be started by activation of an adjustment application or an instruction from a user.

When the change control of the Vol or the EQ is detected by the user's manipulation, the parameters of the Vol or the EQ before the change are stored in the parameter buffers 23 and 27 together with the time stamp. Thereafter, when the end of the parameter change is detected, the changed parameters are also stored in the parameter buffers 23 and 27 together with the time stamp.

At least two parameter sets before and after the change can be stored in the parameter buffers 23 and 27 of each ear. The end of the parameter change may be detected, for example, in a case where there is no manipulation for a predetermined time (e.g., 5 Sec), or the predetermined time may be specified by the user himself/herself, or notification of adjustment completion may be performed by the user's manipulation. Once the parameter adjustment is completed, the buffered set of sound and parameter is input to the feedback acquisition units 22 and 26.

The parameter control unit 28 learns the AI illustrated in FIGS. 6 to 8 and 11 by using the feedback stored in the user feedback DB 24. As a result, the parameter control unit 28 can output the result of the auditory disorder determination illustrated in FIG. 1 to the left ear earphone 1L and the right ear earphone 1R as feedback by the learned AI.

Moreover, the parameter control unit 28 outputs the parameters (e.g., parameters of Vol and EQ) of the hearing signal processing illustrated in FIG. 2 to the left ear earphone 1L and the right ear earphone 1R as feedback and automatically adjusts them by the learned AI, so that it is possible to provide sound that is easy for an auditory disorder user to hear.

Furthermore, in a case where learning can be performed by closed information collection by one user, the parameter control unit 28 can update the AI as "event data" if there is at least a sound signal and user situation data. In addition, even if the device is closed by one user, when a different device is used due to a model change or the like, if the unique parameter for each model is recorded and recorded in the user feedback DB24 in association with the event data, the log data recorded in the different model can be effectively utilized.

For example, in order to be able to calculate the actually reproduced absolute sound pressure level from the level of the waveform indicated by the sound waveform or the feature amount recorded as the event data, model-specific information on an audio device such as the level diagram may be recorded as "device-specific data."

Alternatively, in a case where data of a plurality of users is aggregated and used, it is necessary to integrate the data in different users or different devices. Therefore, in order to utilize data of different devices of different users, all three categories may be recorded in association with each other.

In this case, for example, the auditory disorder determination AI can be constituted by performing clustering in a space having the audiogram information as a feature amount vector (e.g., k-means clustering) and classifying the user into a predetermined number of classes.

Note that the user feedback DB24 (storage device 3) may be provided on a device (including the television 4) other than the terminal device 2 such as a server on a network. Moreover, the sound output device 1 (the left ear earphone 1L and the right ear earphone 1R) and the television 4 may directly communicate with the user feedback DB24 provided on a device other than the terminal device 2, or may communicate via the terminal device 2.

Furthermore, the data collecting system 200 may acquire various data collected by the data collecting system 201 and other data collecting systems to perform the auditory disorder determination, the auditory capacity estimation and the future auditory capacity estimation. Similarly, the data collecting system 201 may acquire various data collected by the data collecting system 200 and other data collecting systems to perform the auditory disorder determination, the auditory capacity estimation and the future auditory capacity estimation. In these cases, data collected by a plurality of data collecting systems may be used in combination. In addition, each data collecting system may acquire the auditory disorder determination result and the auditory capacity estimation results generated by other data collecting systems to perform the auditory disorder determination, the auditory capacity estimation and the future auditory capacity estimation.

Furthermore, in the embodiment described so far, an example has been described in which the sound output device 1 is a hearing aid having a hearing aid function, but the sound output device 1 may be a sound collector having a sound collecting function using a similar procedure. Moreover, an example in which firmware (FW) is updated when the sound output device 1 is made into a hearing aid (sound collector) has been described. However, the hearing aid (sound collector) may be made by using means such as activation of a disabled function instead of the FW update.

In this case, the processing device accepts the selection manipulation corresponding to the notification based on the estimation result of the auditory capacity of the user, and enables the hearing aid function or the sound collecting function of the sound output unit on the basis of the selection manipulation. And the sound output unit performs the hearing aid or the sound collecting process. At this time, the sound output unit performs hearing aid or sound collecting processing on the basis of the hearing aid parameter for adjusting the function of the sound output unit.

Note that the effects described in the present specification are merely examples and are not limited, and other effects may be exerted. Furthermore, in the present embodiment, the case where the terminal device 2 has the functions of the manipulation device and the processing device has been described, but the sound output device 1 may have all the functions of the manipulation device and the processing device.

Note that the present technology can also have the following configurations.
(1)
   1. An information processing method of an information processing system, including:
      an adjustment manipulation accepting step of accepting adjustment manipulation of sound output from a sound output unit; and
      a feedback output step of estimating auditory capacity of a user corresponding to the adjustment manipulation from the adjustment manipulation and signal data of the sound corresponding to the adjustment manipulation, and outputting feedback.
(2) The information processing method according to (1), wherein,
   in the feedback output step, a notification based on an estimation result of the auditory capacity of the user is presented to the user as output of the feedback.
(3) The information processing method according to (1), wherein,
   in the feedback output step,
   a parameter for adjusting a function of the sound output unit is output to the sound output unit as the feedback based on an estimation result of the auditory capacity of the user.
(4) The information processing method according to (3), wherein,
   in the feedback output step,
   a notification for changing the parameter is presented in a case where a value indicating an estimated auditory capacity level of the user is equal to or less than a threshold, and the parameter is changed based on manipulation corresponding to the notification.
(5) The information processing method according to any one of (1) to (4), wherein,
   in the feedback output step,
   the auditory capacity of the user is estimated based on a learning model for estimating the auditory capacity of the user.
(6) The information processing method according to any one of (1) to (5), wherein,
   in the feedback output step,
   the auditory capacity of the user is estimated from volume adjustment manipulation for adjusting volume of the sound or adjustment manipulation of an equalizer for adjusting sound quality of the sound.
(7) The information processing method according to any one of (1) to (6), wherein,
   in the feedback output step,
   the auditory capacity of the user is estimated from a feature amount of the signal data.
(8) The information processing method according to any one of (1) to (7), wherein,
   in the feedback output step,
   latest auditory capacity of the user is estimated based on an estimation result of the auditory capacity of the user estimated in time series.
(9) The information processing method according to any one of (1) to (8), wherein,
   in the feedback output step,
   future auditory capacity of the user is estimated based on an estimation result of the auditory capacity of the user estimated in time series.
(10) The information processing method according to (5), further including
   a data collecting step of collecting event data, which includes manipulation data of the adjustment manipulation by the user, signal data of the sound when the adjustment manipulation is performed and situation data of the user when the adjustment manipulation is performed, device-specific data related to sound output of the sound output unit, and user-specific data, which includes physical data related to the auditory capacity of the user, and storing the event data, the device-specific data, and the user-specific data in association with each other, wherein
   the learning model performs machine learning for an estimation method of the auditory capacity by using data collected in the data collecting step.
(11) The information processing method according to (10), wherein,
   in the data collecting step,
   at least any one of the event data, the device-specific data or the user-specific data is collected, the event data including at least any one of an emotion of the user estimated from an image of the user captured by an imaging device and an environment around the user, the device-specific data including a level diagram of the sound output unit, and the user-specific data including at least any one of an auditory capacity test result, a genetic test result or a blood test result of the user.
(12) The information processing method according to any one of (1) to (11), further including
   a sound output step of outputting the sound by the sound output unit.
(13) The information processing method according to (2), further including:
   a selection manipulation accepting step of accepting selection manipulation corresponding to notification based on an estimation result of the auditory capacity of the user;
   a function enabling step of enabling a hearing aid function or a sound collecting function of the sound output unit based on the selection manipulation; and
   a hearing step or a sound collecting step in which the sound output unit performs hearing or sound collecting processing.
(14) The information processing method according to (3) or (4), further including
   a hearing step or a sound collecting step in which the sound output unit performs hearing or sound collecting processing based on a parameter for adjusting a function of the sound output unit.
(15) An information processing system including:
   a manipulation unit that accepts adjustment manipulation of sound output from a sound output unit; and
   a processing unit that estimates auditory capacity of a user corresponding to the adjustment manipulation from the adjustment manipulation and signal data of the sound corresponding to the adjustment manipulation, and outputs feedback.
(16) The information processing system according to (15), further including
   a sound output unit that outputs the sound.
(17) The information processing system according to (16), wherein
   the sound output unit is an earphone, and
   the manipulation unit and the processing unit are mounted on the earphone or a terminal device that outputs the signal data to the earphone.
(18)
   18. The information processing system according to (16), wherein
   the sound output unit is a speaker of a television, and
   the manipulation unit is a remote control of the television.
(19) A data collecting method including:
   a manipulation data collecting step of collecting manipulation data of a manipulation unit by a user from the manipulation unit that accepts adjustment manipulation of sound output by a sound output unit;
   a sound signal data collecting step of collecting signal data of the sound when the manipulation unit is manipulated from the sound output unit; and
   an event data generating step of generating event data by associating the manipulation data with the signal data; and
   a storing step of storing the event data in a storage unit.
(20) A data collecting system including:
   a sound output unit that outputs sound;
   a manipulation unit that accepts adjustment manipulation of the sound output from the sound output unit; and
   a data collecting unit that collects manipulation data of the manipulation unit by a user of the sound output unit and signal data of the sound when the manipulation unit is manipulated.

### Reference Signs List

100 INFORMATION PROCESSING SYSTEM
1 SOUND OUTPUT DEVICE
2 TERMINAL DEVICE
200, 201 DATA COLLECTING SYSTEM

## Claims

1. An information processing method of an information processing system, comprising:
an adjustment manipulation accepting step of accepting adjustment manipulation of sound output from a sound output unit; and
a feedback output step of estimating auditory capacity of a user corresponding to the adjustment manipulation from the adjustment manipulation and signal data of the sound corresponding to the adjustment manipulation, and outputting feedback.

2. The information processing method according to claim 1, wherein,
in the feedback output step, a notification based on an estimation result of the auditory capacity of the user is presented to the user as output of the feedback.

3. The information processing method according to claim 1, wherein,
in the feedback output step,
a parameter for adjusting a function of the sound output unit is output to the sound output unit as the feedback based on an estimation result of the auditory capacity of the user.

4. The information processing method according to claim 3, wherein,
in the feedback output step,
a notification for changing the parameter is presented in a case where a value indicating an estimated auditory capacity level of the user is equal to or less than a threshold, and the parameter is changed based on manipulation corresponding to the notification.

5. The information processing method according to claim 1, wherein,
in the feedback output step,
the auditory capacity of the user is estimated based on a learning model for estimating the auditory capacity of the user.

6. The information processing method according to claim 1, wherein,
in the feedback output step,
the auditory capacity of the user is estimated from volume adjustment manipulation for adjusting volume of the sound or adjustment manipulation of an equalizer for adjusting sound quality of the sound.

7. The information processing method according to claim 1, wherein,
in the feedback output step,
the auditory capacity of the user is estimated from a feature amount of the signal data.

8. The information processing method according to claim 1, wherein,
in the feedback output step,
latest auditory capacity of the user is estimated based on an estimation result of the auditory capacity of the user estimated in time series.

9. The information processing method according to claim 1, wherein,
in the feedback output step,
future auditory capacity of the user is estimated based on an estimation result of the auditory capacity of the user estimated in time series.

10. The information processing method according to claim 5, further comprising
a data collecting step of collecting event data, which includes manipulation data of the adjustment manipulation by the user, signal data of the sound when the adjustment manipulation is performed and situation data of the user when the adjustment manipulation is performed, device-specific data related to sound output of the sound output unit, and user-specific data, which includes physical data related to the auditory capacity of the user, and storing the event data, the device-specific data, and the user-specific data in association with each other, wherein
the learning model performs machine learning for an estimation method of the auditory capacity by using data collected in the data collecting step.

11. The information processing method according to claim 10, wherein,
in the data collecting step,
at least any one of the event data, the device-specific data or the user-specific data is collected, the event data including at least any one of an emotion of the user estimated from an image of the user captured by an imaging device and an environment around the user, the device-specific data including a level diagram of the sound output unit, and the user-specific data including at least any one of an auditory capacity test result, a genetic test result or a blood test result of the user.

12. The information processing method according to claim 1, further comprising
a sound output step of outputting the sound by the sound output unit.

13. The information processing method according to claim 2, further comprising:
a selection manipulation accepting step of accepting selection manipulation corresponding to notification based on an estimation result of the auditory capacity of the user;
a function enabling step of enabling a hearing aid function or a sound collecting function of the sound output unit based on the selection manipulation; and
a hearing step or a sound collecting step in which the sound output unit performs hearing or sound collecting processing.

14. The information processing method according to claim 3, further comprising
a hearing step or a sound collecting step in which the sound output unit performs hearing or sound collecting processing based on a parameter for adjusting a function of the sound output unit.

15. An information processing system comprising:
a manipulation unit that accepts adjustment manipulation of sound output from a sound output unit; and
a processing unit that estimates auditory capacity of a user corresponding to the adjustment manipulation from the adjustment manipulation and signal data of the sound corresponding to the adjustment manipulation, and outputs feedback.

16. The information processing system according to claim 15, further comprising
a sound output unit that outputs the sound.

17. The information processing system according to claim 16, wherein
the sound output unit is an earphone, and
the manipulation unit and the processing unit are mounted on the earphone or a terminal device that outputs the signal data to the earphone.

18. The information processing system according to claim 16, wherein
the sound output unit is a speaker of a television, and
the manipulation unit is a remote control of the television.

19. A data collecting method comprising:
a manipulation data collecting step of collecting manipulation data of a manipulation unit by a user from the manipulation unit that accepts adjustment manipulation of sound output by a sound output unit;
a sound signal data collecting step of collecting signal data of the sound when the manipulation unit is manipulated from the sound output unit; and
an event data generating step of generating event data by associating the manipulation data with the signal data; and
a storing step of storing the event data in a storage unit.

20. A data collecting system comprising:
a sound output unit that outputs sound;
a manipulation unit that accepts adjustment manipulation of the sound output from the sound output unit; and
a data collecting unit that collects manipulation data of the manipulation unit by a user of the sound output unit and signal data of the sound when the manipulation unit is manipulated.
